# EUROPÄISCHE PATENTSCHRIFT

(11) **EP 1 249 530 B1**
(45) Veröffentlichungstag und Bekanntmachung des Hinweises auf die Patenterteilung: **14.12.2005**
(21) Anmeldenummer: 02007886.1
(22) Anmeldetag: 09.04.2002
(51) Int. Cl.: D06H 3/08, D01G 31/00, G01N 33/36, G01N 21/89

(54) **Sensorleiste**
Sensor array
Réseau lineaire de capteurs

(30) Priorität: 09.04.2001 DE 10117698
(43) Veröffentlichungstag der Anmeldung: 16.10.2002
(73) Patentinhaber: Hergeth, Hubert, Dipl.-Ing., Dipl.-Wirt.-Ing., 6343 Rotkreuz (CH)
(72) Erfinder: Hergeth, Hubert, Dipl.-Ing., Dipl.-Wirt.-Ing., 6343 Rotkreuz (CH)
(74) Vertreter: Cohausz & Florack

(56) Entgegenhaltungen:
- WO-A-95/16909
- CH-A- 654 608
- CH-A- 675 306
- DE-A- 3 412 451
- DE-A- 3 803 353
- DE-A- 4 340 165
- DE-A- 4 340 173
- DE-A- 4 430 332
- DE-A- 19 521 552
- US-A- 4 728 800

## Beschreibung

In der Textilindustrie werden Zeilenkameras verwendet, um Fremdteile in Faserflockenströmen zu erkennen oder Warenbahnen auf Fehler wie Löcher, Dickstellen und Farbabweichungen zu erkennen. In den bekannten Anwendungen werden ein oder mehrere Zeilenkameras verwendet, die etwa 90 ° zur Transportrichtung über die Breite inspizieren.

Nachteilig ist hierbei der durch die Optik bestimmte große Abstand zwischen der Kamera und dem zu inspizierenden Material.

In dem Raum dazwischen kommt es zu Verschmutzungen. Die Distanz führt zu großen maschinenbaulichem Aufwand, und die Justierung der Kameras ist diffizil.

Eine Vorrichtung zum Detektieren von Defekten einer bewegten Stoffbahn durch Detekiereinheiten, die als Module mit Sensoren ausgebildet sind, ist aus US-A-4 728 800 bekannt.

Aufgabe der Erfindung ist es, ein System zu schaffen, das die Erkennung mit erheblich weniger Maschinenbauaufwand und höherer Zuverlässigkeit ermöglicht.

Erfindungsgemäß geschieht dies durch die Verwendung von sehr langen CCD oder CMOS Sensorleisten (z. B. Toshiba), die über die zu inspizierende Breite direkt angeordnet sind.

Die Sensoren sind durch ein oder mehrere Glasscheiben von dem Textilgut geschützt. Die dem Textilgut am nächsten gelegene Glasscheibe wird vom Textilgut selbständig geputzt. Die Sensoren können direkt an der Glasscheibe angebracht werden; es entfällt die Verschmutzungsgefahr und die Maschinen können sehr viel kompakter gebaut werden.

Die Sensorleisten haben sehr viele Sensorpunkte, so daß eine Auflösung von etwa 1/10 mm erreicht werden kann und der elektrische Installationsaufwand gering bleibt.

Es ist nicht notwendig, wie vielfach angenommen, daß das Textilgut in einer durchgehenden Zeile inspiziert werden muß.

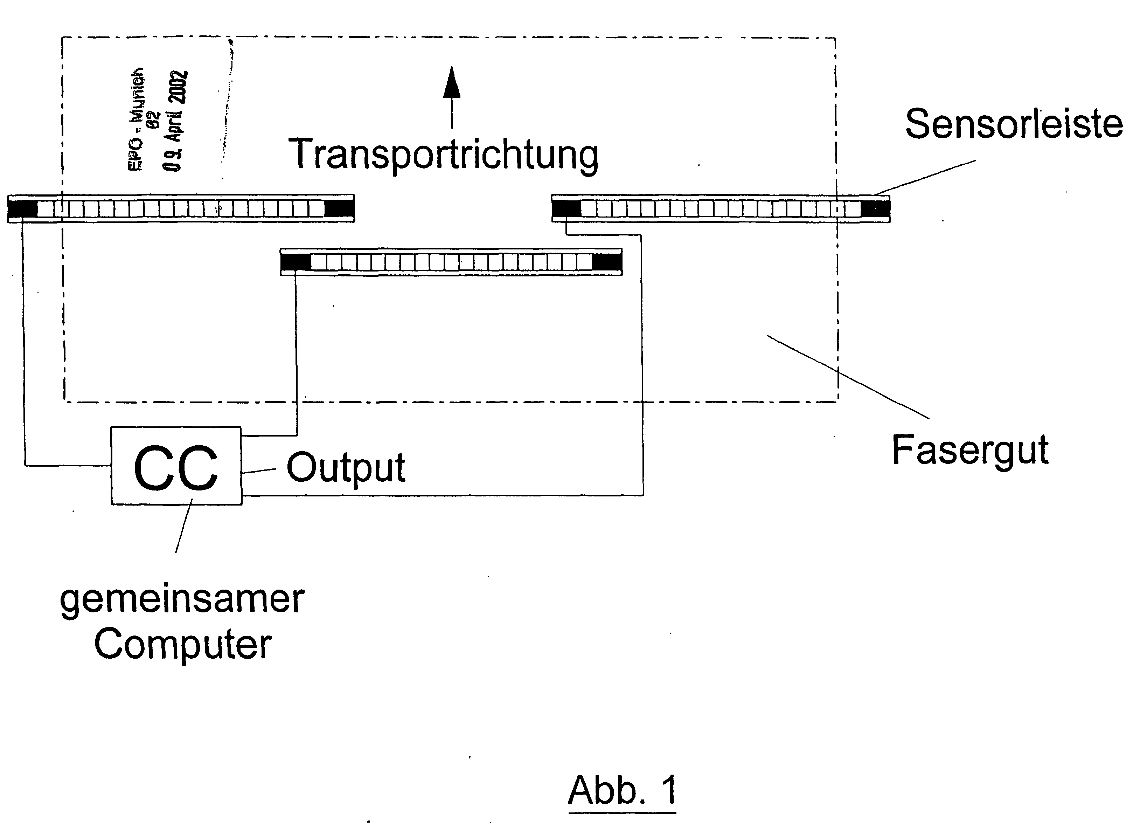

Abbildung 1 zeigt schematisch ein System.

Ein Fasergut, z. B. eine Bahn aus Textilfasern, bewegt sich unter Sensorleisten, die sich über die Breite der Bahn erstrecken, hinweg. Die Sensorleisten sind versetzt angeordnet, da die CCD oder CMOS Elemente am Anfang und Ende nicht mit Photoelementen bestückt sind; so kann die ganze Bahn lückenlos inspiziert werden. Die Sensorleisten sind mit einem gemeinsamen Auswertecomputer verbunden.

## Patentansprüche

1. Sensorsystem zur Inspektion von bewegtem Textilfasergut wobei in etwa 90° zur Transportrichtung des Textilfaserguts mindestens 1 Sensorleisten angebracht ist, **dadurch gekennzeichnet, dass** die mindestens 1 Sensorleiste aus mehreren CMOS oder CCD Elementen gefertigt ist und jedes dieser Elemente mindestens 16 Pixel aufweist.

2. Sensorsystem zur Inspektion von bewegtem Textilgut nach Anspruch 1) **gekennzeichnet**, **daß** in etwa 90° zur Transportrichtung des Textilguts mindestens 3 Sensorleisten versetzt oder gestaffelt angebracht sind und mit einem gemeinsamen Auswertesystem verbunden sind.

3. Sensorsystem nach einem der Ansprüche 1) bis 2) **dadurch gekennzeichnet, daß** die aktiven Sensorflächen durch den Versatz das Textilgut lückenlos inspizieren können.

4. Sensorsystem nach einem der Ansprüche 1) bis 3) **dadurch gekennzeichnet, daß** die Sensorleisten durch die gemeinsame Glasscheibe das Textilgut inspizieren.

5. Sensorsystem nach einem der Ansprüche 1) bis 4) **dadurch gekennzeichnet, daß** die Sensorleiste Farbunterschiede feststellen kann.

6. Sensorsystem nach einem der Ansprüche 1) bis 5) **dadurch gekennzeichnet, daß** das Textilgut die Glastrennwand zwischen Sensorleiste und Textilgut berührt.

7. Sensorsystem nach einem der Ansprüche 1) bis 6) **dadurch gekennzeichnet, daß** die Sensorleisten einzeln austauschbar sind.

## Claims

1. A sensor system for inspecting moved textile fiber material, wherein at least one sensor line is affixed at approximately 90° to the transport direction of the textile fiber material,
**characterized in that** the at least one sensor line is manufactured from multiple CMOS or CCD elements and each of these elements has at least 16 pixels.

2. The sensor system for inspecting moved textile material according to Claim 1,
**characterized in that** at least three sensor lines are affixed offset or stepped at approximately 90° to the transport direction of the textile material and are connected with a joint evaluation system.

3. The sensor system according to one of Claims 1 to 2,
**characterized in that** the active sensor surfaces may inspect the textile material completely due to the offset.

4. The sensor system according to one of Claims 1 to 3,
**characterized in that** the sensor lines inspect the textile material through the joint glass disk.

5. The sensor system according to one of Claims 1 to 4,
**characterized in that** the sensor lines may determine color differences.

6. The sensor system according to one of Claims 1 to 5,
**characterized in that** the textile material touches the glass separating wall between the sensor line and the textile material.

7. The sensor system according to one of Claims 1 to 6,
**characterized in that** the sensor lines are individually replaceable.

## Revendications

1. Système de détecteurs pour l'inspection de produit en fibres textiles en déplacement, au moins une réglette de détecteur étant installée à sensiblement 90° par rapport à la direction du transport du produit en fibres textiles, **caractérisé en ce que** l'au moins une réglette de détecteur est fabriquée à partir de plusieurs éléments CMOS ou CCD et **en ce que** chacun de ces éléments présente au moins 16 pixels.

2. Système de détecteurs pour l'inspection d'un produit en fibres textiles en mouvement, **caractérisé en ce qu'**au moins 3 réglettes de détecteurs sont installées en décalage ou de manière échelonnée à sensiblement 90° par rapport à la direction de transport du produit textile et sont reliées à un système d'évaluation commun.

3. Système de détecteurs selon l'une des revendications 1 ou 2, **caractérisé en ce que** les surfaces actives des détecteurs peuvent effectuer une inspection sans discontinuité grâce au déplacement du produit textile.

4. Système de détecteurs selon l'une des revendications 1 à 3, **caractérisé en ce que** les réglettes de détecteurs inspectent le produit textile à travers la vitre commune.

5. Système de détecteurs selon l'une des revendications 1 à 4, **caractérisé en ce que** les réglettes de détecteurs peuvent constater des différences de couleur.

6. Système de détecteurs selon l'une des revendications 1 à 5, **caractérisé en ce que** le produit textile touche la paroi de séparation en verre entre la réglette de détecteurs et le produit textile.

7. Système de détecteurs selon l'une des revendications 1 à 6, **caractérisé en ce que** les réglettes de détecteurs peuvent être remplacées individuellement.
